# EUROPEAN PATENT APPLICATION

(11) **EP 2 281 811 A1**
(43) Date of publication of application: **09.02.2011**
(21) Application number: 09176522.2
(22) Date of filing: 19.11.2009
(51) Int. Cl.: C07C 237/46, C07C 231/24

(54) **A continuous deacetylation and purification process in the synthesis of non-ionic X-ray contrast agents**

(30) Priority: 21.07.2009 US 227092 P
(71) Applicant: GE Healthcare AS, 0401 Oslo (NO)
(72) Inventor: Askildsen, Arne, 4521 Spangereid (NO); Cervenka, Jan, 0401 Oslo (NO); Hussain, Khalid, 0401 Oslo (NO); Sørensen, Thore, Jarle, 4517 Mandal (NO); Malthe-Sørenssen, Didrik, 4521 Spangereid (NO)
(74) Representative: Wulff, Marianne Weiby

(57) **Abstract**

This invention relates to an improved method for the synthesis of 5-acetamido-N,N'-bis(2,3-dihydroxypropyl)-2,4,6-triiodoisophthalamide ("Compound A"), an intermediate in the industrial preparation of non-ionic X-ray contrast agents. In particular, it relates to a continuous process of deacetylation of the acetylated hydroxyl group in 5-amino-N,N'-bis(2,3-dihydroxypropyt)-2,4,6-triiodo-1,3-benzenedicarboxarriide ("Compound B") followed by crystallisations, filtration, and washing of Compound A.

## Description

### TECHNICAL FIELD

This invention relates generally to large-scale synthesis of non-ionic X-ray contrast agents. It further relates to an improved method for the synthesis and purification of 5-acetamido-N,N'-bis(2,3-dihydroxypropyl)-2,4,6-triiodoisophthalamide ("Compound A"), an intermediate in the industrial preparation of non-ionic X-ray contrast agents. In particular, it relates to a continuous process for deactylation of the acetylated hydroxyl groups and subsequent purification of Compound A.

### BACKGROUND OF THE INVENTION

Non-ionic X-ray contrast agents constitute a very important class of pharmaceutical compounds produced in large quantities. 5-[N-(2,3-dihydroxypropyl)-acetamido]-N,N'-bis(2,3-dihydroxypropyl)-2,4,6-triiodo-isophthalamide ("iohexol"), 5-[N-(2-hydroxy-3-methoxypropyl)acetamido]-N,N'-bis(2,3-dihydroxypropyl)-2,4,6-triiodo-isophthalamide ("iopentol"), and 1,3-bis(acetamido)-N,N'-bis[3,5-bis(2,3-dihydroxypropyl-aminocarbonyl)-2,4,6-triiodophenyl]-2-hydroxypropane ("iodixanol") are important examples of such compounds. They generally contain one or two triiodinated benzene rings.

For example, iodixanol, marketed under the trade name Visipaque®, is one of the most used agents in diagnostic X-ray procedures. It is produced in large quantities by GE Healthcare in Lindesnes, Norway. The industrial production of iodixanol involves a multistep chemical synthesis as shown in Scheme 1 below. *See also* U.S. Patent No. 6,974,882. To reduce the cost of the final product, it is critical to optimize each synthetic step. Even a small improvement in reaction design can lead to significant savings in a large scale production.

In the acetylation step of the industrial scale synthesis 5-amino-N,N'-bis(2,3-dihydroxypropyl)-2,4,6-triiodoisophthalamide (Compound B), is acetylated to produce 5-acetylamino-N,N'-bis(2,3-dihydroxypropyl)-2,4,6-triiodoisophthalamide (Compound A) using acetic anhydride as the acetylating reagent. The instant invention is directed to a continuous process for deactylation of the undesired acetylated groups generated during the acetylation step and subsequent purification of Compound A.

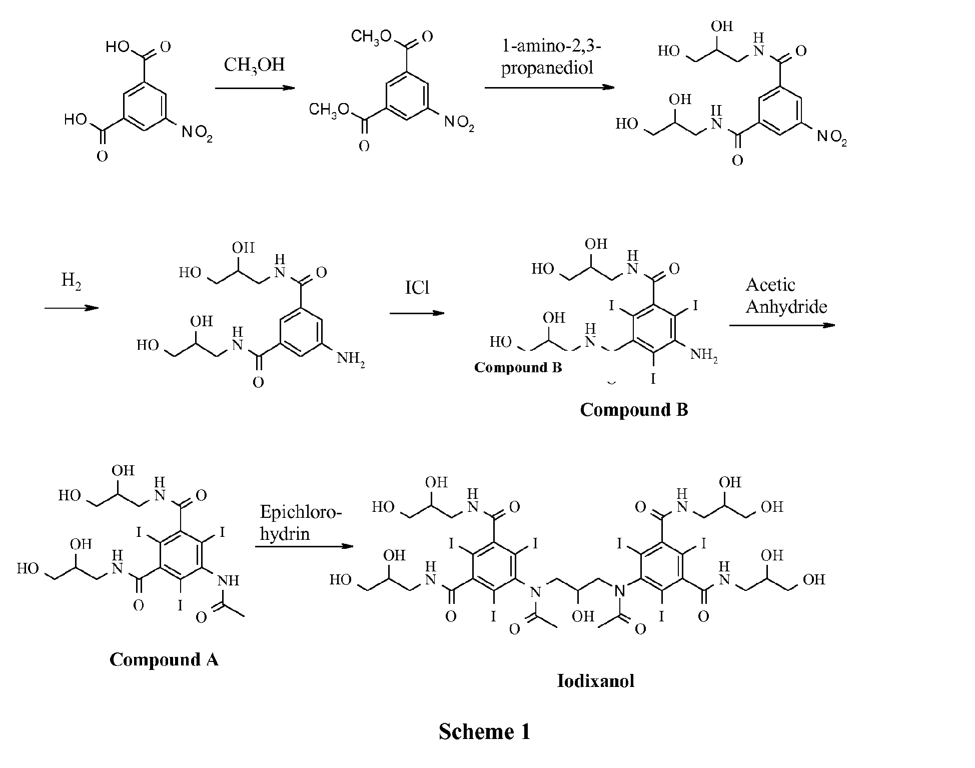

### BRIEF DESCRIPTION OF THE DRAWINGS

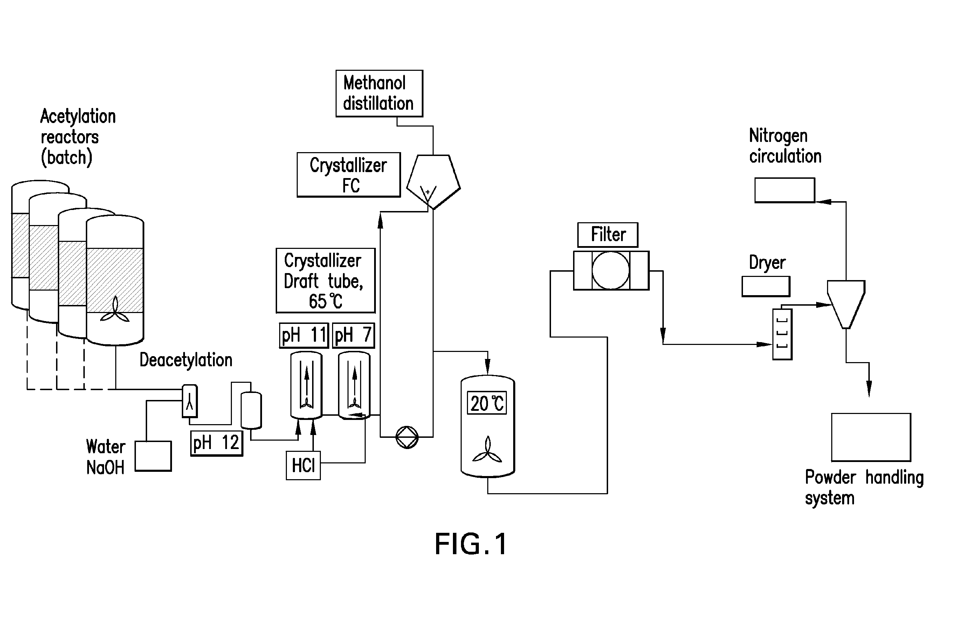

**FIG. 1** is a schematic diagram of one exemplary embodiment of the instant invention.

### SUMMARY OF THE INVENTION

The present invention provides an industrial process for purifying Compound A. Specifically, it uses a continuous process comprising the steps of (1) deacetylating the acetylated hydroxyl groups of 5-amino-N,N'-bis(2,3-dihydroxypropyl)-2,4,6-triiodoisophthalamide ("Compound B") in a first reactor at a pH between about 11 and about 12 by adding aqueous sodium hydroxide or another suitable base; (2) crystallising Compound A in a second reactor at a pH between about 11 and about 12 at a temperature of between about 10°C and about 70 °C; (3) crystallising Compound A in a third reactor at a pH between about 6 and about 8 and at a temperature higher than or equal to about 10°C and lower than or equal to that in step (2); and (4) filtering and washing the crystals of Compound A.

A reactor in the instant process may be a tubular flow reactor, a continuous stirred tank reactor (CSTR), or other suitable continuous reaction equipment.

### DETAILED DESCRIPTION OF THE INVENTION

In a batch process, all the operations are performed successively in the same reactor. Thus, production capacity increase in a batch production requires very large reactor volumes and a corresponding increase in capital investments. On the other hand, a continuous process has dedicated equipment for every operation, where the mixture moves from one operation to the next within the production line. All the operations are performed continuously all the time and the system is at a steady state. Consequently, a continuous production process requires much smaller equipment volumes and investments for achieving the same production capacity. In addition, a continuous operation allows for less varying quality of the product. While desirable, a continuous system is more complicated to design and is highly specific to one single product.

In the acetylation reaction of Compound B, both the amino group and the four hydroxyl groups are acetylated. *See* Scheme 2. The acetylated hydroxyl groups are undesirable and thus deacetylated following the acetylation reaction.

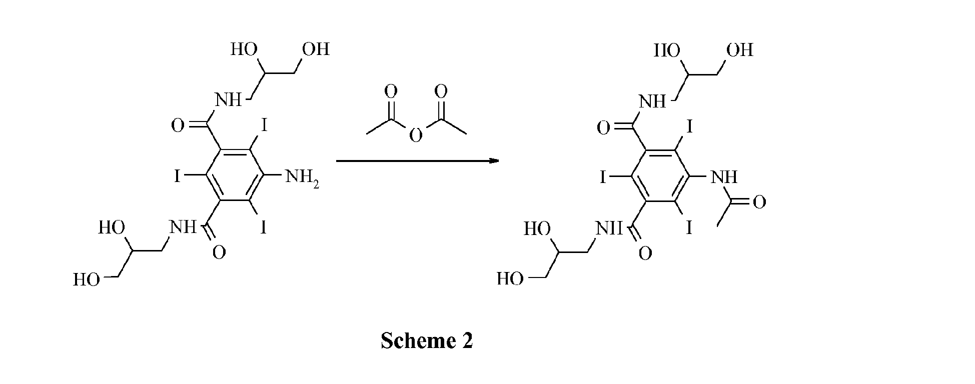

In the present invention, we have found that the process from deacetylation to dry Compound A can be run continuously using a number of sequential steps. First, the undesired acetylated hydroxyl group is deacetylated at a pH between about 11 and about 12 by adding a base such as aqueous sodium hydroxide. Under this condition, only the desired - NH-Ac group survives the hydrolysis and remains acetylated. Preferably, the concentration of NaOH used is about 10-50 w/w %, more preferably 20-25 w/w %. The reaction is strongly exothermic. Sodium acetate is formed as a byproduct.

Second, Compound A is first crystallized at a pH between about 11 and 12 and at a temperature of between about 10°C and about 70 °C in a second reactor.

Third, Compound A is again crystallized in a third reactor at a pH between about 6 and 8, but at a lower or same temperature than the previous crystallisation step. Preferably, the pH is adjusted to about 7 by acid treatment, resulting in precipitation of Compound A. The purity and yield are influenced by the presence of sodium acetate and methanol. High supersaturation is useful for agglomeration of the crystalline product and thus its filterability.

Optionally, crystallisation of Compound A may be divided into more than two steps, each preferably at a different pH, different temperature, and/or different residence time. Preferably, the solvent used may be removed in one of the crystallisation steps, which may increase the yield of Compound A recovery. For example, a first crystallisation step may be conducted at (1) a pH of about 11.0-11.5, preferably about 11.3, (2) a temperature of about 50-65°C, preferably about 60°C, and (3) a residence time of about 1-4 hours, preferably about 2 hours. A second crystallisation step may be conducted at (1) a pH of about 6-8, preferably about 7, (2) a temperature of 50-65°C, preferably about 60°C, and (3) a residence time of about 1-4 hours, preferably about 2 hours. A third crystallisation step may be conducted at (1) a pH of about 6-8, preferably about 7, (2) a temperature of 50-65°C, preferably about 60°C, and (3) a residence time of about 1-4 hours, preferably about 2 hours, simultaneously stripping off up to about 40 % of the mother liquor volume, preferably 15-30 % of the volume. A fourth crystallisation step may be conducted at (1) a pH of about 6-8, preferably about 7, a temperature of about 15-30°C, preferably about 15-20°C, and a residence time of 6-12 hours, preferably about 8 hours.

As the final step of the instant continuous process, Compound A crystals are filtered and washed. Preferably, following filtration and washings, crystals of Compound A are dried. The filtration, washing and drying steps may preferably be combined in one step using a suitable combined filter dryer.

The instant process provides Compound A with consistent quality and yield. Steady state conditions are obtained in the process.

The invention is illustrated further by the following examples that are not to be construed as limiting the invention in scope to the specific procedures described in them.

### EXAMPLES

### EXAMPLE 1

Compound B was acetylated in a mixture of acetic anhydride and acetic acid in the presence of catalytic amounts of p-toluene sulphonic acid at 50-125°C. Excess acetic anhydride and acetic acid was distilled off under reduced pressure, and the reaction mixture was diluted with methanol and water. The resulting reaction mixture had the following composition:

| | |
|---|---|
| Overacetylated Compound A (a mixture of compounds with varying number of acetyl groups attached): | 97.7-98.3 % by area in HPLC |
| Compound B: | 0.0-0.3 % by area in HPLC |
| Concentration: | 31 w/v % Compound A in an approximately 2.5:1 methanol/water mixture |
| Conductivity: | 5.1-5.7 mS/cm |

Compound B was produced from the reaction mixture in a system according to Figure 1. The reaction mixture from the acetylation reaction (prepared batch wise) was fed into the static mixer in a flow ratio of 1.04 mL reaction mixture: 1.0 mL 20 w/w % NaOH at about 55°C (outlet temperature). The absolute feeding rate of the reaction mixture was 2.6 kg/hour. The pH was kept at about 12.2 at steady state. HPLC of the output stream showed Compound A in 98.2 % purity with 0.23 % Compound B present at steady state.

The continuous crystallisation was performed as described above. The pH in the first and second crystalliser varied between 11.1 and 11.5 over time, while the pH in the third and fourth crystalliser varied between 6.2 and 7.2. The temperature was 60°C in the two first crystallisers and 20°C in the third and fourth. Residence times were held at 2, 2, 2 and 8 hours, respectively. 20 % of the mother liquor volume was stripped off in the third crystalliser at a pressure of 240-250 mbar. The water content in the methanolic distillate from the stripping was 34-35 w/w %. The resulting slurry from the fourth crystalliser was transferred to a holding tank before filtration.

The slurry was filtered in a continuous rotation filter at 1.6-3.2 barA. The filtration rate varied between about 500 and 900 L/m2/hour (about 500-650 L/m2/hour at steady state). The filter cake was washed with methanol (about 1.9 kg/kg Compound A) in the same filter. The resulting salt content in the filter cake was 0.2-0.3 w/w % NaCl.

The moist filter cake was dried in a continuous fluid bed (spin flash) dryer at 120-130°C gas temperature. The maximum temperature exposed to the product was 80°C. The resulting moisture content was 0.4 w/w %.

The purity of dry Compound A in HPLC was 99.5 % by area.

All patents, journal articles, publications and other documents discussed and/or cited above are hereby incorporated by reference.

## Claims

1. A process for industrial preparation of 5-acetamido-N,N'-bis(2,3-dihydroxypropyl)-2,4,6-triiodoisophthalamide ("Compound A") comprising the following sequential and continuous steps:
(1) deacetylating the acetylated hydroxyl group of 5-amino-N,N'-bis(2,3-dihydroxypropyl)-2,4,6-triiodoisophthalamide ("Compound B") in a first reactor at a pH between about 11 and about 12 by adding a base;
(2) crystallising Compound A in a second reactor at a pH between about 11 and about 12 at a temperature of between about 10 °C and about 70°C;
(3) crystallising Compound A in a third reactor at a pH between about 6 and about 8 and at a temperature higher than or equal to about 10°C and lower than or equal to that in step (2); and
(4) filtering and washing the crystals of Compound A.

2. The process of Claim 1 further comprising the step of drying the crystals of Compound A.
